# EUROPEAN PATENT SPECIFICATION

(11) **EP 3 655 385 B1**
(45) Date of publication and mention of the grant of the patent: **07.07.2021**
(21) Application number: 18739880.5
(22) Date of filing: 19.07.2018
(51) Int. Cl.: C07C 41/54, C07C 43/303, C07C 403/08, C07C 403/10, C07D 309/12

(54) **PROCESS OF PRODUCTION OF 3,7-DIMETHYL-9-(2,6,6-TRIMETHYL-1-CYCLOHEXEN-1-YL)-NONA-2E,7E-DIEN-4-YNE-1,6-DIOL**
VERFAHREN ZUR HERSTELLUNG VON 3,7-DIMETHYL-9-(2,6,6-TRIMETHYL-1-CYCLOHEXEN-1-YL)-NONA-2E,7E-DIEN-4-YNE-1,6-DIOL
PROCÉDÉ DE PRODUCTION DE 3,7-DIMÉTHYL-9-(2,6,6-TRIMÉTHYL-1-CYCLOHEXÈN-1-YL)-NONA-2E,7E-DIEN-4-YNE-1,6-DIOL

(30) Priority: 20.07.2017 EP 17182235
(43) Date of publication of application: 27.05.2020
(73) Proprietor: DSM IP Assets B.V., 6411 TE Heerlen (NL)
(72) Inventor: BONRATH, Werner, 4303 Kaiseraugst (CH); KUENZI, Rolf, 4303 Kaiseraugst (CH)
(74) Representative: Kurt, Manfred
(86) International application number: PCT/EP2018/069627
(87) International publication number: WO 2019/016314

(56) References cited:
- CH-A- 481 088
- US-A- 2 451 739

## Description

The present invention relates to an improved method for producing 3,7-dimethyl-9-(2,6,6-trimethyl-1-cyclohexen-1-yl)-nona-2E,7E-dien-4-yne-1,6-diol.

3,7-Dimethyl-9-(2,6,6-trimethyl-1-cyclohexen-1-yl)-nona-2E,7E-dien-4-yne-1,6-diol, which trivial name is oxenine, has the following formula (I) 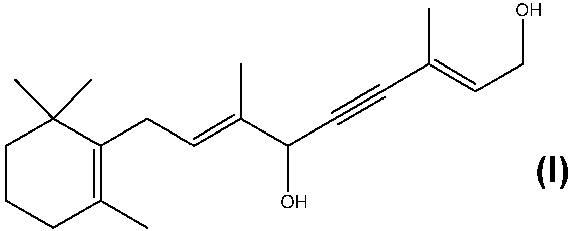 is an intermediate in a process to produce vitamin A (and its derivatives as i.e. vitamin A acetate).

Oxenine as an intermediate in the production of vitamin A is known for a long time. It is known i.e. from Isler et al., Helv. Chim. Acta 30, 1911 (1947); from US 2451739 or from US 3046310.

CH 481088 discloses a coupling reaction of an aldehyde and an acetal intermediate by using lithium amide in liquid ammonia.

### Vitamin A

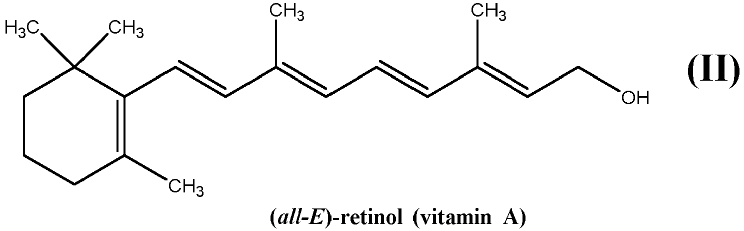

is an important ingredient for many applications. Vitamin A (and its derivatives) plays a role in a variety of functions throughout the (human and animal) body, such as e.g. vision process, gene transcription, immune function, bone metabolism, haematopoiesis, skin and cellular health and antioxidant function.

An important derivative of vitamin A is the vitamin A acetate, which is the compound of the following formula (II'):

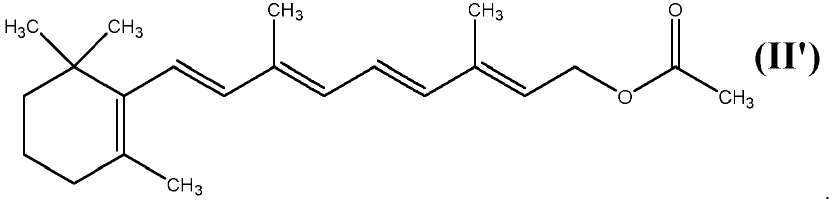

Due to the importance of vitamin A (and its derivatives) and the complexity of the synthesis thereof, there is always a need for improved processes of its production.

Vitamin A (retinol or its derivatives) can be produced (when starting from oxenine, which is the compound of formula (I)) according to the following reaction schemes, which are known i.e. from US2451739:

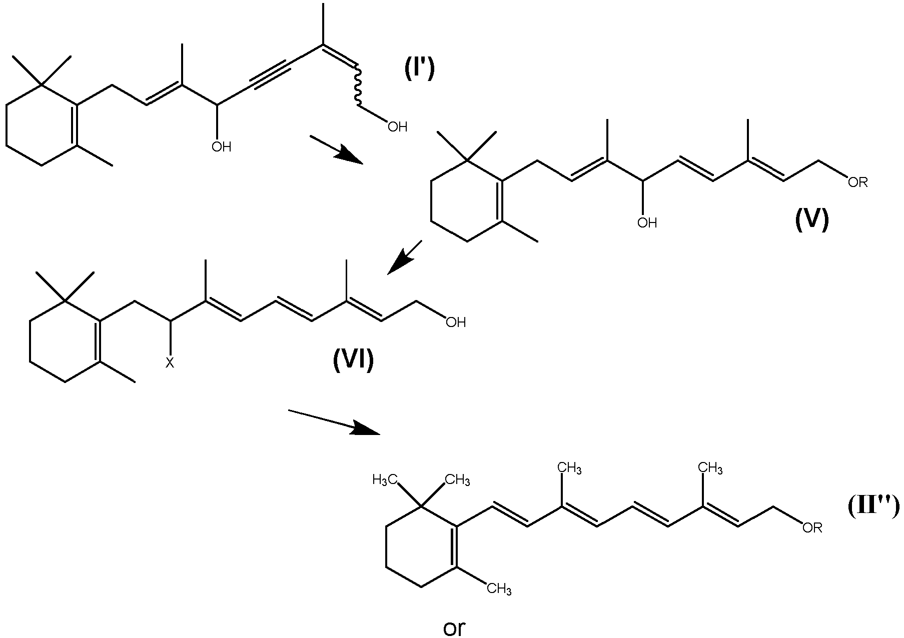

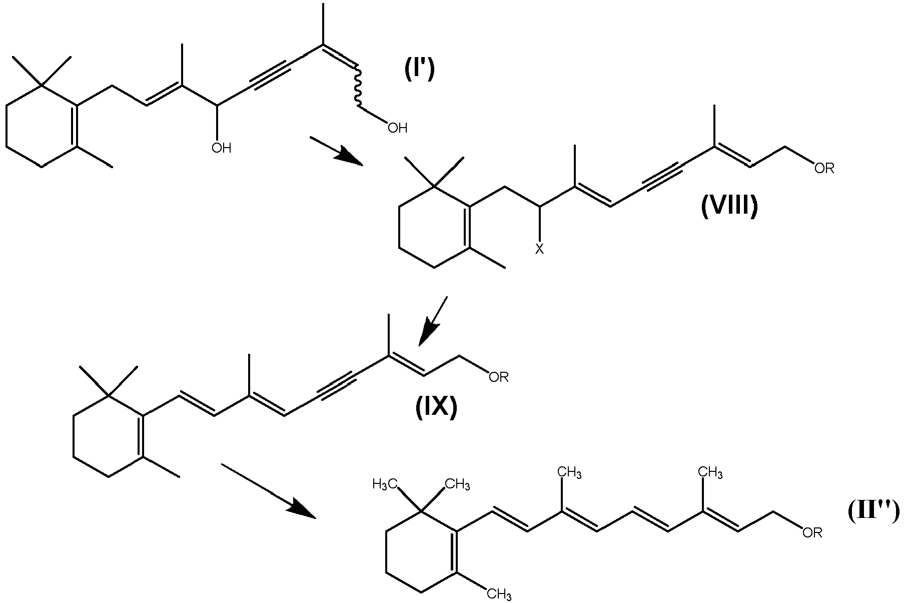

Oxenine is usually obtained by the condensation reaction of the following compound of formula (III) and (IV)

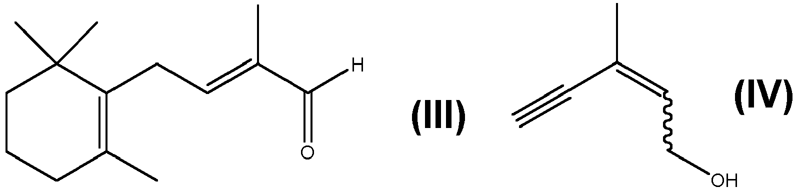

This condensation reaction is usually carried out as a Grignard reaction, wherein a compound of formula (III) 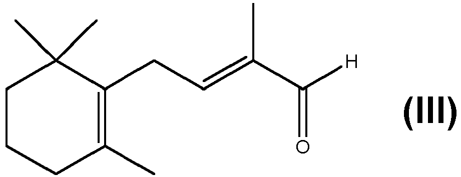 is reacted with the Grignard compound of formula (IV')

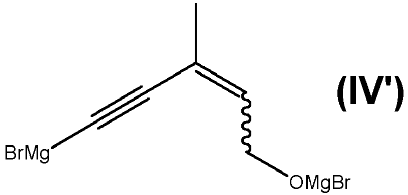

In the prior art, a yield of oxenine of about 50 % is achieved.

Surprisingly, a new process was found wherein the yield of oxenine is improved significantly.

The new process is characterized in that in step (a) the compound of formula (IV), which is (E)-1-pentol (also known as (E-3-methylpent-2-en-4-yn-1-ol) 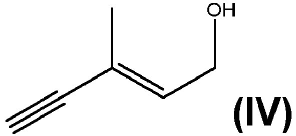 is reacted with a compound of formula (X)

R₁-O-R₂ **(X)**,

wherein
R₁ is a C₁-C₄ alkyl group and
R₂ is a C₃-C₆ alkylene group, or
R₁ and R₂ form together a C₅-C₇ aliphatic ring, comprising at least one C=C bond, and
afterwards in step (b) the reaction product of step (a) is reacted with a compound of formula (XI) 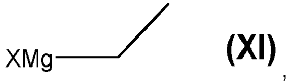 wherein
   X is Cl, Br, or I (preferably Br), and
   finally, in step (c), the reaction product of step (b) is reacted with a compound of formula (III) 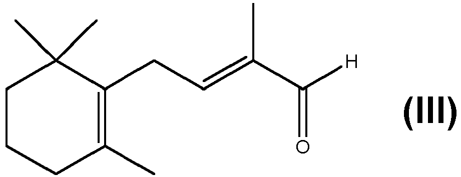 to obtain oxenine (compound of formula (I))

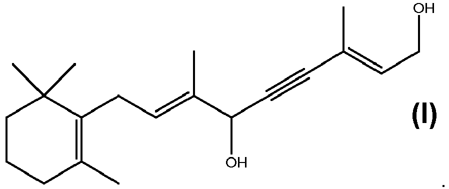

An essential feature of this process is that in step (b) and in step (c) the reactions are carried out in 2-methyltetrahydrofuran (also known as 2-methyloxolane) as a solvent.

2-Methyltetrahydrofuran is the compound of formula (XII)

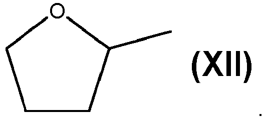

The fact that we found that for step (b) and (c) the same solvent (2-methyltetrahydrofuran) can be used it a surprising and huge advantage. In contrast to the prior art the new process is less work intensive and less material is consumed.

Furthermore, no alkali metals are needed for the process according to the present invention.

Therefore, the present invention relates to a process (P) to produce a compound of formula (I) 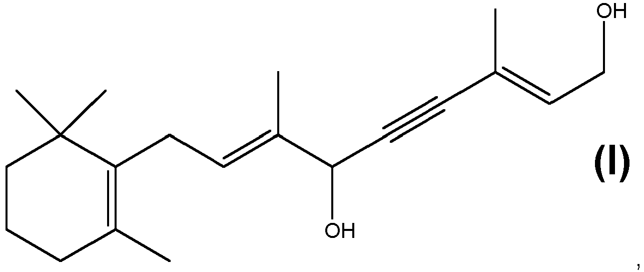 wherein a first step (a)
a compound of formula (IV) 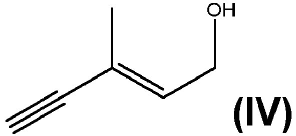 is reacted with a compound of formula (X)

R₁-O-R₂ **(X)**,

wherein
R₁ is a C₁-C₄ alkyl group and
R₂ is a C₃-C₆ alkylene group, or
R₁ and R₂ form together a C₅-C₇ aliphatic ring, comprising at least one C=C bond, and
the reaction product of step (a) is reacted
in a second step (b) with a compound of formula (XI) 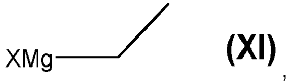 wherein
   X is Cl, Br, or I (preferably Br), and
   in step (c) the reaction product of step (b) reacted with a compound of formula (III) 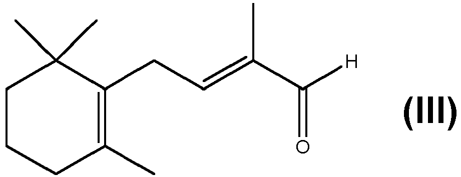
   to form the compound of formula (I) and
   wherein
   the reactions of step (b) and of step (c) are carried out in 2-methyltetrahydrofuran as solvent.

Furthermore. the present invention relates to a process (P'), which is process (P), wherein no alkali metal is used in any process steps of the process according to the invention.

In the following, the 3 steps (a), (b) and (c) are discussed in more details.

### Step (a)

In a first step (step (a)) the compound of formula (IV), 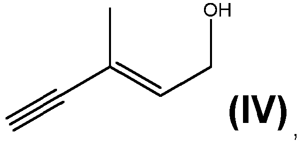 which is (E)-1-pentol, is reacted with the compound of formula (X)

R₁-O-R₂ **(X)**,

wherein
R₁ is a C₁-C₄ alkyl group and
R₂ is a C₃-C₆ alkylene group, or
R₁ and R₂ form together a C₅-C₇ aliphatic ring, comprising at least one C=C bond.

A preferred embodiment of the present invention is a process wherein R₁ is -CH₃ or -CH₂CH₃ and R₂ is C₃-C₅ alkylene group.

Another preferred embodiment of the present invention is a process, wherein R₁ and R₂ together form a C₆ aliphatic ring comprising at least one C=C bond.

A more preferred embodiment of the present invention is a process wherein the compound of formula (X) is a compound of formula (X'), (X") or (X'") 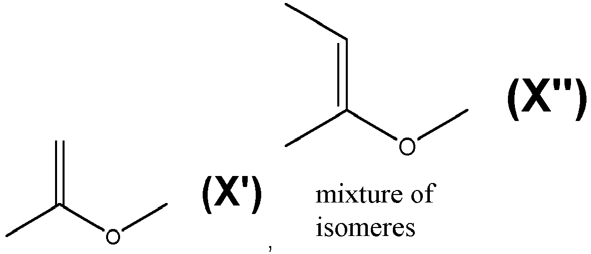 or

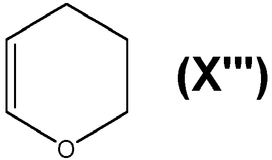

The two starting materials can be added in equimolar ratios.
Usually the compound of formula (X) is added in excess.
Usually the molar ratio of compound (IV) to compound (X) is 1:1 up to 1:4.

The reaction of step (a) is usually (and preferably) catalyzed by at least one acid. The acid can be any commonly used acid.
Very preferred acids are i.e. sulfuric acid, p-toluene sulfonic acid hydrate (pTsOH) or benzoic acid.

The acid is used in a catalytic amount. Usually in an amount which is about 0.001 - 0.0000 1 mol equivalent (in regard to compound of formula (IV)).
Instead of using at least one acid, it is also possible to use an ion exchanger to catalyse the reaction. Strongly acidic cation exchange resins are usually used. Such acidic cation exchangers are available commercially.
Suitable ion exchangers are i.e. Amberlyst 15®, Amberlyst 36®, Amberlyst 70® and Dowex 50 WX12®.

A base is added at the end of the reaction to stop. Any commonly known base (or a mixture of bases) can be used.

The reaction of step (a) is exothermic. Therefore, the reaction mixture is usually cooled. This is done by any usually used external cooling systems.
The reaction of step (a) can be carried out without any solvent.
Alternatively, the reaction of step (a) can be carried out with at least one solvent. Suitable solvents are polar aprotic solvents such as ethers.

### Step (b)

In a second step (step (b)) the reaction product of step (a) is reacted with a compound of formula compound of formula (XI) 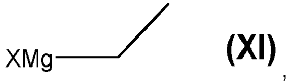 wherein X is Cl, Br, or I (preferably Br).

Compound of formula (XI) is a classical Grignard compound. This compound is usually produced in situ by the addition of magnesium and bromoethane. This is the common way of preparing this compound. The reaction condition for preparing the compound of formula (XI) are the commonly used ones.

The reaction product of step (a) is added slowly to the reaction solution in which the compound of formula (XI) was produced.

2-Methyltetrahydrofuran is used in step (b) as a solvent.

The reaction of step (b) is usually carried out at elevated temperature. Usually at a temperature which is between 30 - 50°C.

The following compounds are the reaction products for the process when the compounds of formula (X'), (X") or (X'") have been used. Dimer compounds are formed when compounds of formula (X') or (X") are used:

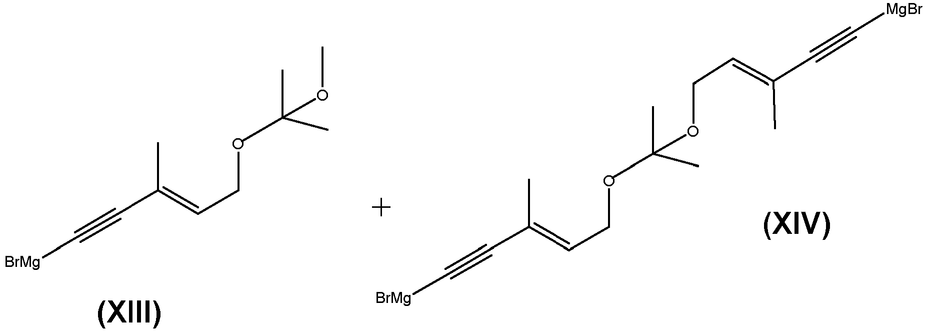

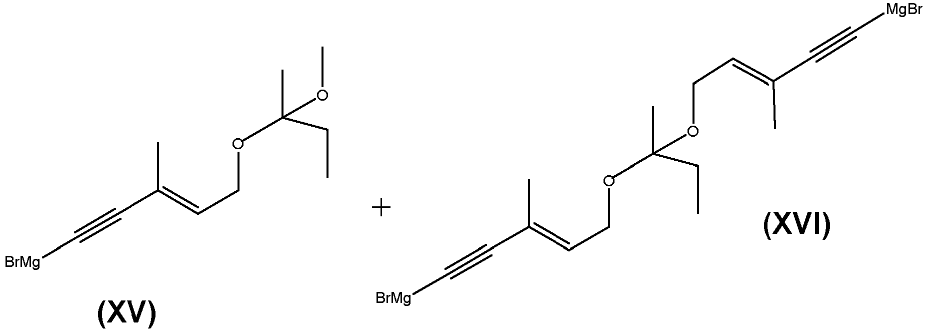

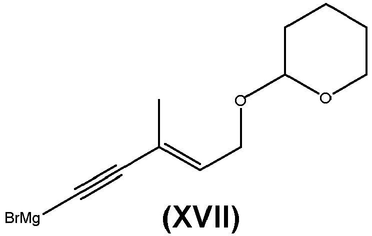

### Step (c)

In a third step (step (c)) the compound of formula (III), 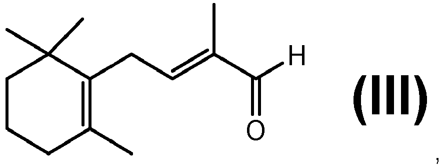 is added to the reaction mixture of step (b).

The reaction of step (c) is usually carried out at elevated temperature. Usually above 30°C up to about 40°C.
An essential feature of the new and improved process of the present invention is that the reactions of steps (b and c) is carried out in 2-methyltetrahydrofuran as solvent.

Some of the so obtained (hydrolyzed not acidified) reaction products are not known yet.
The following compound of formula (XV') is new

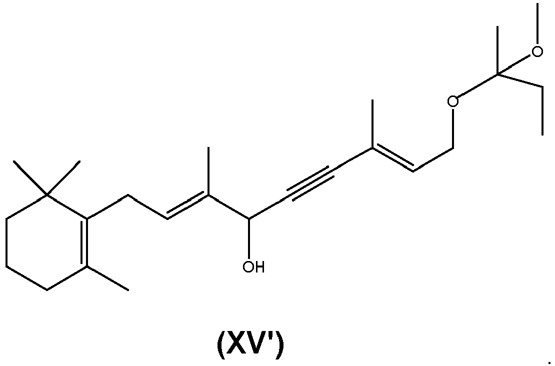

At the end of the reaction, the reaction mixture is poured into an ice/water solution and then this solution is acidified.

An extraction process is applied to isolate the reaction product. The yield of the new process is significantly higher as the yield from the reaction from the prior art.

The following examples serve to illustrate the invention. If not otherwise stated, the temperature is given in degree Celsius and all parts are related to the weight.

### Examples

### Example 1:

6.22 g of (E)-1-pentol (95.8 w%; 62.0 mMol) and 1.35 mg of p-TsOH hydrate (99 %, 7.00 µMol, were charged into a vessel and cooled to -3°C.
Afterwards 10.79 g of isopropenylmethyl ether (14.2 ml, 97 %, 145 mMol) were added dropwise while the temperature of the reaction mixture was kept at 0°C and the reaction mixture was stirred for additional 30 minutes.
9.72 mg Triethylamine (99.8 %, 0.096 mMol) were added and the reaction mixture was heated to about 20°C.
The non-reacted starting material was removed under reduced pressure (about 27 °C and up to 30 mbar).
The so obtained reaction product ((E)-1-pentol/isopropenylmethyl ether-product) was dissolved in 8 ml of 2-methyltetrahydrofuran (water-free).

1.36 g of magnesium (99.95 %, 56.0 mMol), in the form of flakes were charged in another vessel under argon.
2.7 ml of 2-methyltetrahydrofuran (water-free) were added to the magnesium.

6.79 g of ethyl bromide (99.7 %, 62.1 mMol) were dissolved in 3.4 ml of 2-methyltetrahydrofuran (water-free) and a bit of this mixture was added to the magnesium to start the reaction and afterwards another 8 ml of 2-methyltetrahydrofuran (water-free) were added. The rest of the ethyl bromide (in 2-methyltetrahydrofuran) was added slowly. The reaction mixture was always kept at a temperature of between 35 - 37°C.

Afterwards the reaction mixture was kept at temperature of 36 - 38°C under stirring for additional 60 minutes. Then all the magnesium was dissolved and the reaction mixture is slightly greyish and clear (Grignard-solution).

In a next step (step (b)) the (E)-1-pentol/isopropenylmethyl ether-product was added dropwise to the Grignard solution. The temperature of the reaction mixture was kept at a temperature of between 35 - 40 °C.
The reaction mixture was stirred at that temperature for about an hour.

C₁₄-aldehyde, 10.53 g of the (compound of formula (III)) (98 w%, 50 mMol,) was dissolved in 5.4 ml of 2-methyltetrahydrofuran (water-free) and afterwards added to the solution of step (b) dropwise. The reaction temperature was kept at a temperature of between 35 - 40 °C.
The reaction mixture was stirred at a temperature of between 35 - 40 °C for about an hour, cooled to room temperature (20 - 25°C), and added to 67 g of a water/ice mixture under stirring.

To this mixture about 21 g of a 15 w% H₂SO_{4aq} were added and the pH was adjusted to pH 1.25 - 1.3 (measured by 780 pH meter of Metrohm (using an electrode 6.99104.02).
The mixture was stirred for about 2 hours at room temperature.

Afterwards ether was added to the reaction mixture and washed with a saturated NaHCO_{3aq} solution.

The organic phase was dried and the solvent was removed under reduced pressure (0.1 mbar at 40 °C).

The yield of the final and pure product (oxenine) was 89 %.

In comparison, the prior art the yield was improved significantly.

Further examples were carried out wherein p-TsOH was replaced by an ion exchanger.
Furthermore, isopropenylmethyl ether was also replaced by butenylmethyl ether and also by 3,4-dihydro-2H-pyran. The reaction conditions were the same as in Example 1.
The obtained yields were very good (in the same range as in Example 1).

The yields of oxenine of the new and improved process according to the present invention were improved significantly.

## Claims

1. A process to produce a compound of formula (I) 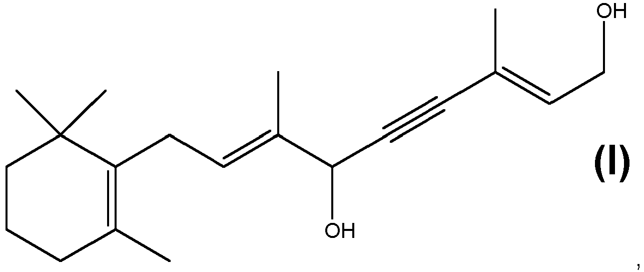 wherein a first step (a)
a compound of formula (IV) 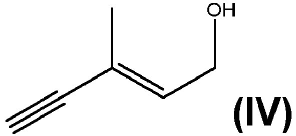 is reacted with a compound of formula (X)
R₁-O-R₂ **(X)**,
wherein
R₁ is a C₁-C₄ alkyl group and
R₂ is a C₃-C₆ alkylene group, or
R₁ and R₂ form together a C₅-C₇ aliphatic ring, comprising at least one C=C bond, and
the reaction product of step (a) is reacted
in a second step (b) with a compound of formula (XI) 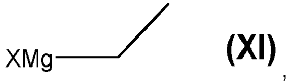 wherein
X is Cl, Br, or I (preferably Br), and
in step (c) the reaction product of step (b) reacted with a compound of formula (III) 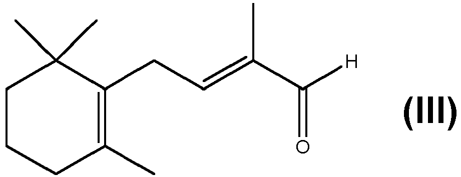
to form the compound of formula (I), and
wherein
the reactions of step (b) and of step (c) are carried out in 2-methyltetrahydrofuran.

2. Process according to claim 1, wherein the reaction of step (a) is catalyzed by at least one (organic) acid.

3. Process according to claim 2, wherein the acid is chosen from the group consisting of sulfuric acid, p-toluene sulfonic acid hydrate and benzoic acid.

4. Process according to claim 1, wherein the reaction of step (a) is catalyzed by an acidic cation exchanger.

5. Process according to anyone of the preceding claims, wherein compound of formula (X) is a compound of selected from the group consisting of 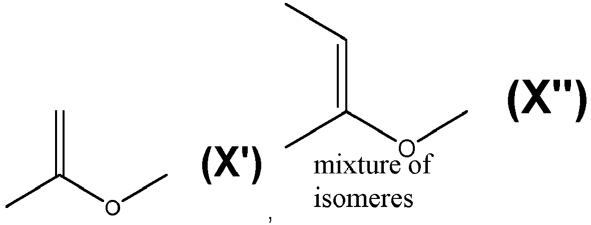 and

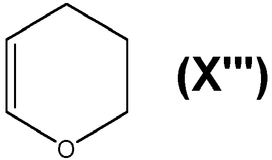

## Patentansprüche

1. Verfahren zur Herstellung einer Verbindung der Formel (I) 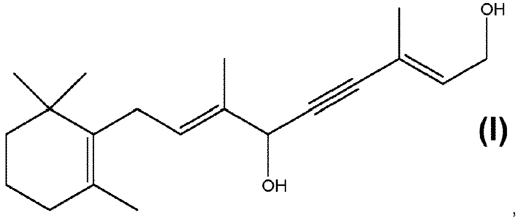 wobei in einem ersten Schritt (a)
eine Verbindung der Formel (IV) 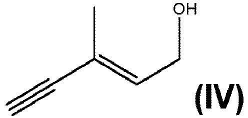 mit einer Verbindung der Formel (X)
R₁-O-R₂ **(X)**,
wobei
R₁ für eine C₁-C₄-Alkylgruppe steht und
R₂ für eine C₃-C₆-Alkylengruppe steht oder
R₁ und R₂ zusammen einen aliphatischen C₅-C₇-Ring mit mindestens einer C=C-Bindung bilden,
umgesetzt wird und
das Reaktionsprodukt von Schritt (a)
in einem zweiten Schritt (b) mit einer Verbindung der Formel (XI) 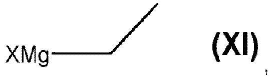
wobei X für Cl, Br oder I (vorzugsweise Br) steht, umgesetzt wird und
in Schritt (c) das Reaktionsprodukt von (b) mit einer Verbindung der Formel (III) 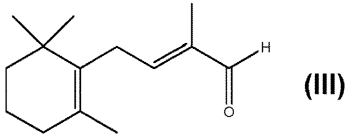
umgesetzt wird, unter Bildung der Verbindung der Formel (I), und
wobei
die Reaktionen von Schritt (b) und Schritt (c) in 2-Methyltetrahydrofuran durchgeführt werden.

2. Verfahren nach Anspruch 1, wobei die Reaktion von Schritt (a) durch mindestens eine (organische) Säure katalysiert wird.

3. Verfahren nach Anspruch 2, wobei die Säure aus der aus Schwefelsäure, p-Toluolsulfonsäure-hydrat und Bezoesäure bestehenden Gruppe ausgewählt ist.

4. Verfahren nach Anspruch 1, wobei die Reaktion von Schritt (a) durch einen sauren Kationenaustauscher katalysiert wird.

5. Verfahren nach einem der vorhergehenden Ansprüche, wobei es sich bei der Verbindung der Formel (X) um eine aus der aus 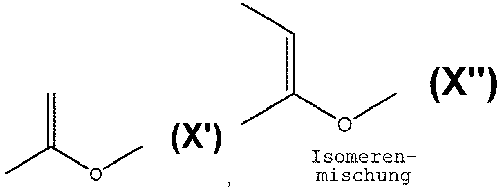 und 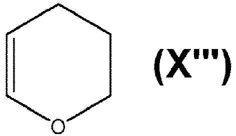 bestehenden Gruppe ausgewählte Verbindung handelt.

## Revendications

1. Procédé pour la production d'un composé de formule (I) 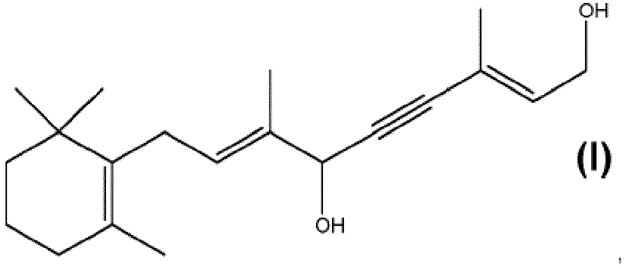 dans une première étape (a)
un composé de formule (IV) 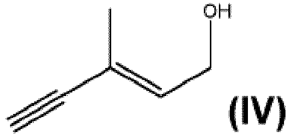 est mis à réagir avec un composé de formule (X)
R₁ O-R₂ **(X)**,
R₁ étant un groupe C₁₋₄ alkyle et
R₂ étant un groupe C₃₋₆ alkylène, ou
R₁ et R₂ formant ensemble un cycle aliphatique en C₅₋₇, comprenant au moins une liaison C=C, et
le produit de réaction de l'étape (a) étant mis à réagir
dans une deuxième étape (b) avec un composé de formule (XI) 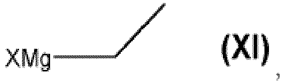
X étant Cl, Br, ou I (préférablement Br), et
dans l'étape (c) le produit de réaction de l'étape (b) étant mis à réagir avec un composé de formule (III) 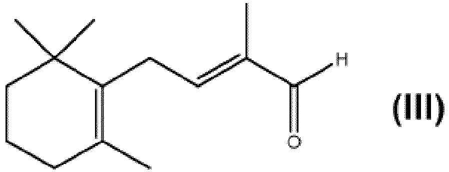
pour former le composé de formule (I), et
les réactions de l'étape (b) et de l'étape (c) étant mises en œuvre dans le 2-méthyltétrahydrofuranne.

2. Procédé selon la revendication 1, la réaction de l'étape (a) étant catalysée par au moins un acide (organique).

3. Procédé selon la revendication 2, l'acide étant choisi dans le groupe constitué par l'acide sulfurique, l'hydrate d'acide p-toluènesulfonique et l'acide benzoique.

4. Procédé selon la revendication 1, la réaction de l'étape (a) étant catalysée par un échangeur de cations acide.

5. Procédé selon l'une quelconque des revendications précédentes, le composé de formule (X) étant un composé choisi dans le groupe constitué par 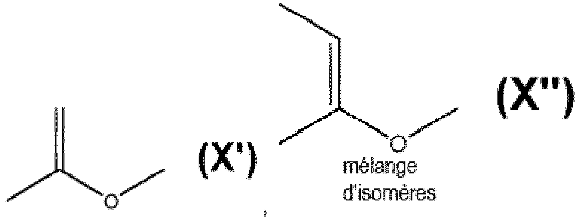 et
